# EUROPEAN PATENT APPLICATION

(11) **EP 1 721 602 A1**
(43) Date of publication of application: **15.11.2006**
(21) Application number: 05010202.9
(22) Date of filing: 11.05.2005
(51) Int. Cl.: A61K 9/08, A61K 31/192, A61K 31/196, A61K 31/19, A61K 31/195, A61K 31/545, A61K 31/546

(54) **Controlled release oral drug delivery system**

(71) Applicant: The Jordanian Pharmaceutical Manufacturing Co., 11710 Naor (JO)
(72) Inventor: Badman, Adnan, Dr., 11185 Amman (JO); Al-Remawi, Mayyas, Dr., 13713 Russiefa (JO)
(74) Representative: Winkler, Andreas Fritz Ernst

(57) **Abstract**

The invention relates to a molecular ionic complex formed between an acidic pharmaceutical drug or its salt or solvate and glucosamine, chondroitin, hyaluronic acid or heparin or its salts or derivatives. The complex can provide for a liquid oral controlled drug delivery. For example, ibuprofen sodium complex with glucosamine sulfate potassium (GSP) was found to have a zero order controlled drug delivery when given orally to rabbits.

## Description

### Field of Invention

This invention relates to a pharmaceutical composition comprising an ionic complex of an acidic drug as an active ingredient and a complex-forming compound consisting of one of glucosamine or its salt, derivative or polymer and/or a similar compound such as chondroitin sulfate, hyaluronic acid and heparin sulfate and/or their derivatives as safe materials for complexation. Further optionally, the composition contains one or more of water-soluble and/or water-dispersible diluents so that the therapeutically active ingredient is released at a rate suitable for once or twice daily.

The invention covers liquid dosage forms either in form of a clear solution or in a suspension form. However, other pharmaceutical dosage forms known in the art can be also included such as intravenous, intramuscular and subcutaneous injectables; creams, gels and suppositories as semisolid dosage forms; tablets and capsules as solid dosage forms.

### Background of Invention

Most oral drugs used to treat diseases are given more than once during a dosage regimen. These products are called immediate release (IR). In order to decrease dose frequency many formulas were invented to be given once per day, these formulas are called controlled release (CR). Other advantages of formulating CR products are to decrease drug plasma level fluctuation and to increase patient convenience. This has been achieved by the development of new drug delivery systems utilizing diverse techniques and principles.

However, most oral products known in the art to control drug release were formulated as solid dosage forms. The present invention, for the first time, presents a liquid controlled release dosage form through complex formation of an acidic drug with glucosamine or a similar compound.

Glucosamine is an amino monosaccharide found in chitin, glycoproteins and glycosaminoglycans (formerly known as mucopolysaccharides) such as hyaluronic acid and heparin sulfate. Glucosamine is also known as 2-amino-2-deoxyglucose, 2-amino-2-deoxy-beta-D-glucopyranose and chitosamine. Glucosamine has the following chemical structure:

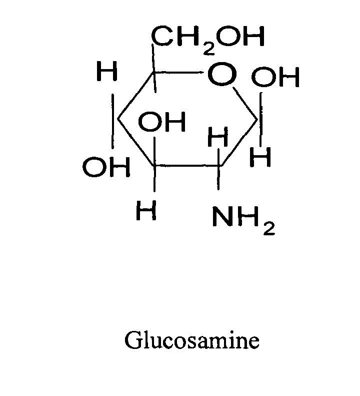

Glucosamine is available commercially as a nutritional supplement in three forms: glucosamine hydrochloride, glucosamine sulfate and N-acetyl-glucosamine. At neutral as well as physiological pH, the amino group in glucosamine is protonated, resulting in its having a positive charge. Salt forms of glucosamine contain negative anions to neutralize the charge. N-acetylglucosamine is a delivery form of glucosamine in which the amino group is acetylated, thus neutralizing its charge. Glucosamine used in supplements is typically derived from marine exoskeletons. Synthetic glucosamine is also available (Glucosamine effects in humans: a review of effects on glucose metabolism, side effects, safety considerations and efficacy. J.W. Anderson, R.J. Nicolosi, J.F. Borzelleca. Food and Chemical Toxicology 43 (2005) 187-201).

To date, most of the clinical studies examining the effect of glucosamine have been performed with either the sulfate or the chloride salts of glucosamine. All three forms are water-soluble. Glucosamine has an effect in relieving joint pain associated with osteoarthritis. The combination of ibuprofen (nonsteroidal anti-inflammatory drug NSAID) and glucosamine is known in the art to be useful in the provision of immediate relief of the symptoms associated with arthritis and similar disorders and injuries which are manifest by swelling and inflammation of joints, and the connective tissues within the joints. The aim of addition of glucosamine with NSAID drugs in the prior art is to have a combined synergistic increase in the therapeutic and/or to decrease the dose of NSAIDs (Nutraceuticals as therapeutic agents in osteoarthritis. The role of glucosamine, chondroitin sulfate and collagen hydrolysate. Deal CL, Moskowitz RW. Rheum Dis Clin North Am. 1999; 25:379-395; Evaluation of glucosamine sulfate compared to ibuprofen for the treatment of temporomandibular joint osteoarthritis: a randomized double blind controlled 3 month clinical trial, Thie N M; Prasad N G; Major P W Orofacial Pain Clinic, Department of Dentistry, Faculty of Medicine and Dentistry, University of Alberta, Edmonton, Alberta, Canada. Journal of Rheumatology (2001 Jun), 28(6), 1347-55; Efficacy and safety of glucosamine sulfate versus ibuprofen in patients with knee osteoarthritis. Qiu G X; Gao S N; Giacovelli G; Rovati L; Setnikar I Peking Union Medical College Hospital, The People's Republic of China Arzneimittel-Forschung (1998 May), 48(5), 469-74; Glucosamine sulfate compared to ibuprofen in osteoarthritis of the knee. Muller-Fassbender H; Bach G L; Haase W; Rovati L C; Setnikar I Rheumazentrum, Bad Abbach, Germany. Osteoarthritis and cartilage / OARS, Osteoarthritis Research Society (1994 Mar), 2(1), 61-9; Antinociceptive synergy, additivity, and subadditivity with combinations of oral glucosamine plus nonopioid. Analgesics in mice. Ronald J. Tallarida, Alan Cowan, Robert B. Raffa. The Journal of Pharmacology and Experimental Therapeutics. Vol. 307 (2003), No. 2, 699-704).

However, in our invention the aim of the addition of a salt of an acidic drug such as NSAID and glucosamine salt is to have a complex that is used to control the drug release in liquid dosage form.

Due to the cationic nature of water-soluble glucosamine salt and the anionic nature of salts of acidic drugs, a simple combination between these salts in aqueous medium results in the formation of a complex, in the pH range 5.0-7.5. This complex was tested in vivo for drug release property and it showed a controlled release liquid dosage form. This may be due to high affinity of the drug to the complex and the delay in drug dissociation from the complex.

### Summary of Invention

Disclosed herein is a composition comprising an ionic complex formed between an acidic pharmaceutical agent, or a salt or solvate thereof, and at least one complex-forming compound selected from the group consisting of glucosamine, chondroitin, hyaluronic acid and heparin, or a salt or derivative of one of these. The ionic complex resulting from, for example, glucosamine and ibuprofen has a controlled release behavior with zero order kinetics in an in vivo test. This complex is dissociated regularly in a zero order pattern inside a biological system. Thus, the advantage of the system over other traditional dosage forms is the decrease in the side effects of the active molecules through the decrease in drug fluctuation inside plasma. Another important effect is to decrease drug dose frequency. The most important advantage over prior art is that the present invention can deliver the drug in a controlled release oral liquid dosage form to be given easily to infants and children in solution form. However, this complex can be given as a tablet, or suppository, or cream or injectable or any pharmaceutical dosage form known in the art.

### Detailed Description of Invention

Compositions of the invention comprise glucosamine and/or its salts or derivatives and/or similar compounds such as chondroitin sulfate and/or its derivatives in combination with an acidic drug molecule or its salts such as ibuprofen, diclofenac sodium, naproxen, sodium valproate etc. In an embodiment of the present invention the mole ratio of complex-forming compound to acidic drug ranges 1:10 to 10:1, preferably 1:1.

Typically the amount of glucosamine or chondroitin sulfate administered per day ranges from 500 mg to about 2500 mg. Acidic drugs such as ibuprofen can be administered in combination with the aforementioned agents to obtain an ionic salt of increased benefit, for example, with stronger medical effects, extended drug release and less side effects. With regard to arthritis and inflammatory joint diseases, these diseases are chronic and need long time of treatment. Thus, it is of importance to decrease drug side effects, maximize cure rates, and decrease dose frequencies to have a good drug regimen.

Compositions can be administered by a variety of methods, which are known in the art. Routes of administration include, oral, topical, intranasal, intraocular, intravenous, intramuscular, transdermal and also by inhalation.

For the purpose of the invention, a suitable vehicle is used to deliver the complex. Any standard pharmaceutical acceptable vehicle known in art can be used. For example, in case of delivery of solution dosage forms distilled water, phosphate buffers of variable pH ranges, normal saline, phosphate saline containing wetting agents, cosolvents, simple syrup, flavoring agent, coloring agents, suspending agents and preservatives can be used.

Examples of suitable vehicles for solid dosage forms (capsules or tablets) include fillers, glidants, binders, disintergrants, and lubricants.

Examples of active agents for use with the invention are: beta lactam antibiotics such as cephalosporins and penicillins; quinolone antibiotics such as nalidixic acid, ciprofloxacin; sulfonamide such as acediasulfone; nonsteroidal anti-inflammatory drugs such as amfenac, diclofenac, ibuprofen, acetyl salicylic acid, clinadac, naproxen, mefenamic acid, fosfomycin, bendazac; antihyperlipidimic such as acifran, fluvastatin, atorvastatin; psychostimulants such as aceglutamide; prostaglandin vasodilators such as alprostadil; antidepressant such as amineptine, gamma aminobutyric acid, gabapentin; anticonvulsive agents such as valproic acid; hemostatic agents such as aminocaproic acid; antituberculosis agents such as aminosalicylic acid; hepatic protectant amino acid arginine, aspartic acid, betaine; flavoring agents and sweeteners such as aspartame; antispasmodic agents such as baclofen; antineoplastic agents such as bendamustine, bromebric acid, sodium borocaptate, chlorambucil, methotrexate; antiseptics such as bismuth subgallate, dodicin; antiinflammatory cortisone drugs such as betamethasone; hypoglycemic oral drugs such as calcium mesoxalate; antiarrythmetic agents such as capobenic acid; antihypertesive agents such as captopril; antiallergy agents such as cromolyn sodium, spaglumic acid; emollient such as docusate sodium. GI disorder agents such as mesalamine; and antinfectives such as flumequine

### Example 1: Preparation of liquid controlled release system from ionic complex between glucosamine sulfate potassium and ibuprofen sodium

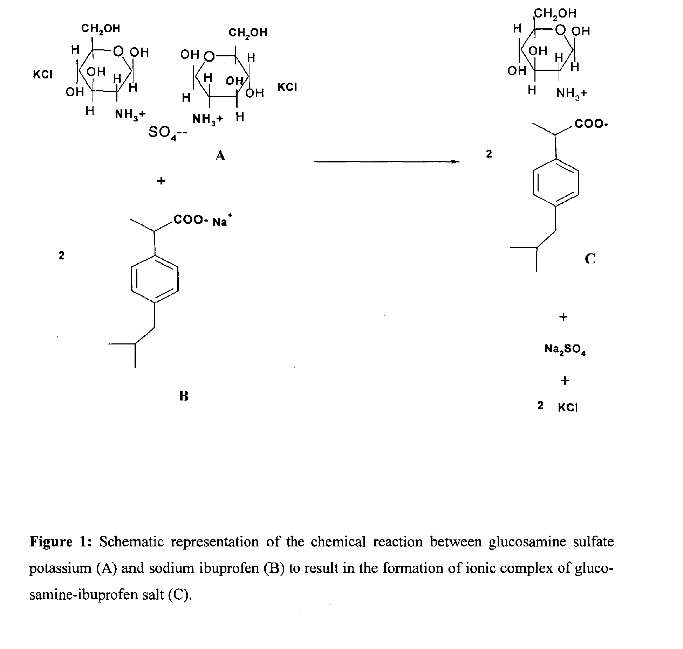

1 mole of sodium ibuprofen dissolved in water (pH around 7) and 1 mole of glucosamine (pH around 5) were reacted. The two solutions were added to each other and the pH was adjusted to be (6.8-7.0). The solutions were stirred for 24 hr before use. Since ibuprofen sodium is a salt of a weak acid and glucosamine sulfate is a salt of a weak base, a salt formation between them is expected as depicted in Fig 1.

### Example 2: In vivo testing liquid controlled release system of ibuprofen -glucosamine ionic complex (prepared in example 1) when given as oral solution to rabbits

The ionic complex containing ibuprofen and glucosamine oral solutions was given orally to rabbits in a dose of 30 mg of ibuprofen/kg. A reference immediate release oral solution given with a dose of 10 mg of ibuprofen/kg rabbit. Blood samples were withdrawn at specified time interval, centrifuged at 3500 rpm for 10 minutes and placed in a freezer at -20°C. The samples were analyzed using HPLC method. A simple rapid method of determining the ibuprofen concentration by HPLC was developed. Naproxen was used as an internal standard. *Mobile Phase:* Water: Acetonitrile (40:60), then adjust pH to 2.4 with H₃PO₄. *Column:* Waters, Symmetry, 5u, C18, and 150*4.6 mm. *Internal STD Stock Solution:* Dissolve 5 mg of Naproxen into 50 ml MeOH. *Injection Loop:* 50 µl. *Flow rate:* 1 ml/min. *Wavelength:* 220 nm.

Sample Preparation: Transfer 100 µl of plasma sample to test tube, add 10 µl of Internal STD Stock solution, add 0.25 ml of 1 M HCl, shake for 30 seconds, add 5 ml of (85:15)(Hexane: Isopropanol), shake with vortex for 1 min., centrifuge at 3000 rpm for 10 min., transfer 4 ml of organic layer to new test tube, evaporate the organic solvent using an air shower, and reconstitute with 1 ml mobile phase. The method was evaluated for specificity showing that there is no interference with the ibuprofen peak. Recovery was 85-90% for ibuprofen. The calibration curve was linear over the concentration 0.5-10 ug/ml.

The ionic complex solution contains 12 mg ibuprofen/ml. The dose of the drug given to each rabbit was 30 mg/kg.

The immediate release aqueous solution contains ibuprofen sodium with a concentration of 4 mg ibuprofen/ml. The dose of the drug given as a reference was 10 mg/kg. This dose (10 mg/kg rabbit) is equivalent to 600 mg ibuprofen given to a human subject weighted 60 kg (The usual human immediate release dose for adults is 200-600 mg given 3 times per day). In the controlled release preparation the formula should contain total daily dose and to be given once i.e. 30 mg/kg.

This justifies the dose of the controlled release solution being three times more than the immediate release one. One of the advantages of controlled release is to decrease the frequency of drug dosing by giving the dose one time per day.

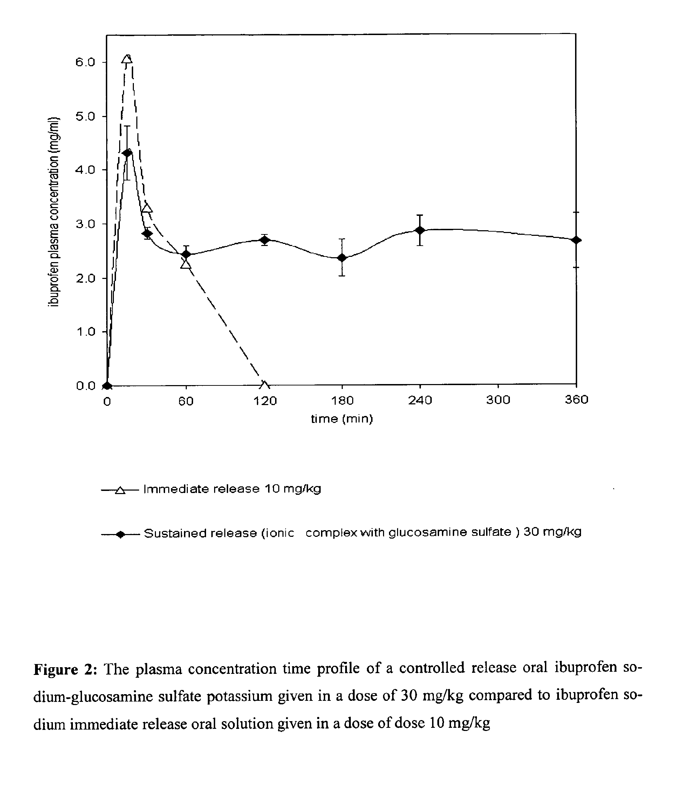

The drug release from the immediate release occurs very quick, reaches a maximum and also declines very quick, while drug release from the ionic complex was zero-order pH independent as shown in Fig. 2.

This clearly indicates that the 30 mg/kg sustained release dose is not absorbed rapidly. Small fractions of the drug werea delivered as the complex transfer through out the GIT independing on the pH changes that occur inside the gut.

Above results illustrate that ibuprofen shows a zero order drug release as it passes the gastrointestinal tract when given as a ionic complex with glucosamine.

### Example 3: Preparation of liquid controlled release system from ionic complex between glucosamine sulfate potassium and diclofenac potassium

1 mole of diclofenac potassium dissolved in water and 1 mole of glucosamine were reacted. The two solutions were added to each other and the pH was adjusted to 6.8-7.0. The solutions were stirred for 24 before use. Since diclofenac potassium is a salt of a weak acid and glucosamine sulfate is a salt of a weak base, a salt formation between them the positively charged glucosamine and negatively charged diclofenac is expected.

### Example 4: In vivo testing of liquid controlled release system of diclofenac glucosamine complex (prepared in example 3) when given as oral solution to rabbits

The ionic complex containing diclofenac potassium and glucosamine oral solutions (prepared in example 3) was given orally to rabbits in a dose of 2 mg of diclofenac potassium/kg. A reference immediate release oral solution given with a dose of 1 mg of diclofenac potassium/kg rabbit. Blood samples were withdrawn at specified time interval, centrifuged at 3500 rpm for 10 minutes and placed in a freezer at -20 °C. The samples were analyzed using HPLC method. A simple rapid method of determining the diclofenac plasma concentration by HPLC was carried out (A rapid and sensitive high performance liquid chromatographic method for the determination of diclofenac sodium in serum and its use in pharmacokinetic studies. El-Sayed, Y. M., Abdel-Hamid, M. E., Suleiman, M.S., Najib, N.M., J. Pharm. Pharmacol., 1988, 40, 727-729).

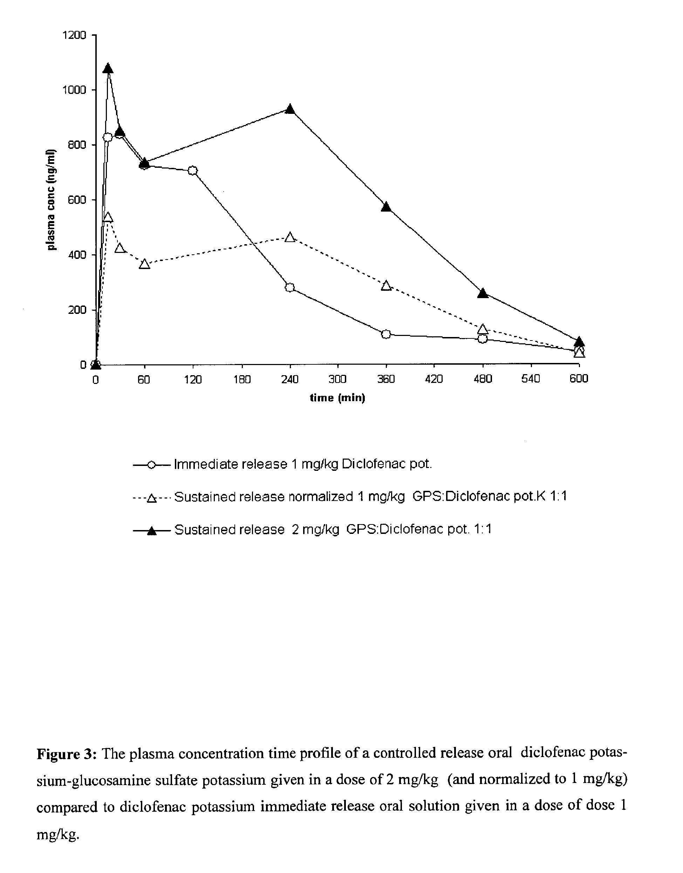

The ionic complex solution dose of the drug given to each rabbit was 2 mg/kg. While, the immediate release aqueous solution dose of the drug given as a reference was 1 mg/kg. This dose (1 mg/kg rabbit) is equivalent to 75 mg diclofenac potassium given to a human subject weighted 75 kg. In the controlled release preparation the formula should contain total daily dose and to be given once i.e. 150 mg. This justifies the dose of the controlled release solution being two times more than the immediate release one. The drug release from the immediate release occurs very quick, reaches a maximum and also declines very quick, while drug release from the ionic complex was zero-order pH independent for a longer period of time (Fig. 3).

### Example 5: Preparation of liquid controlled release system from sodium valproate glucosamine complex in 1:1 molar ratio

1 mole of sodium valproate dissolved in water (pH around 7.31) and 1 mole of glucosamine sulfate potassium (pH 4.60) were reacted. The two solutions were added to each other and the pH was 6.21. The solutions were stirred for 24 before use. A clear liquid solution was obtained which means the complex has a high water-solubility property. Consequently , the complex can be used in oral liquid controlled release formulations.

### Example 6: Preparation of liquid controlled release system from gabapentin glucosamine complex in 1:1 molar ratio

1 mole of gabapentin sodium dissolved in water and 1 mole of glucosamine were reacted. The two solutions were added to each other and the pH was adjusted around 7. The solutions were stirred for 24 before use. A clear liquid solution was obtained which means the complex has a high water-solubility property. Consequently, the complex can be used in oral liquid controlled release formulations.

### Example 7: Preparation of liquid controlled release from cephalexin glucosamine complex in 1:1 molar ratio

1 mole of cephalexin sodium dissolved in water (pH 7.3) were reacted and 1 mole of glucosamine (pH 4.5). The two solutions were added to each other and the pH was 6.8. The solutions were stirred for 24 h before use. A clear liquid solution was obtained which means the complex has a high water-solubility property. Consequently, the complex can be used in oral liquid controlled release formulations.

### Example 8: Preparation of liquid controlled release from cefixime glucosamine complex in 1:1 molar ratio

1 mole of cefixime disodium dissolved in water (pH 7.12) and 1 mole of glucosamine sulfate potassium (pH 4.57) were reacted. The two solutions were added to each other and the pH was 6.5. The solutions were stirred for 24 h before use. A clear liquid solution was obtained which means the complex has a high water-solubility property. Consequently, the complex can be used in oral liquid controlled release formulations.

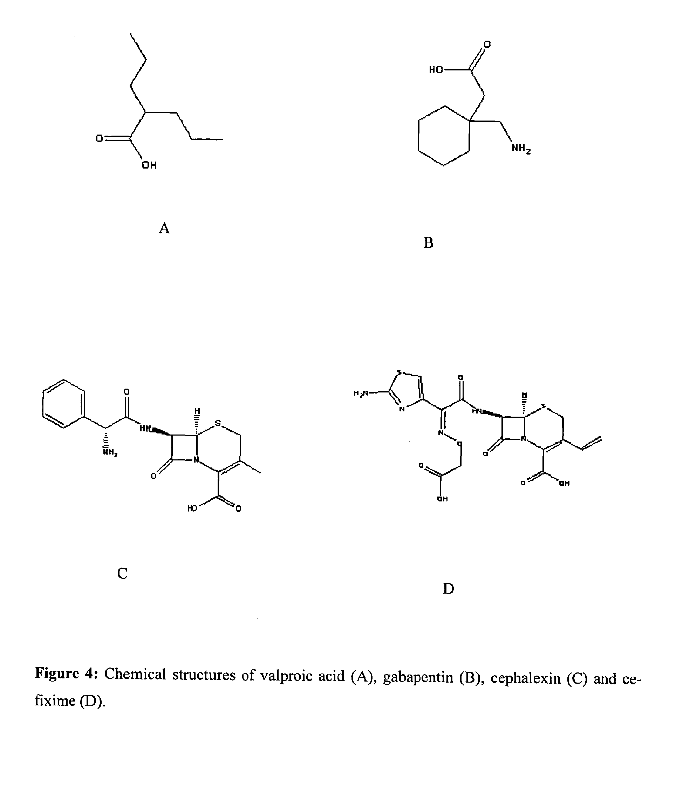

Chemical structures of drugs mentioned in examples 5-8 are shown in Fig 4. Those drugs each contain an acidic funtional group (carboxylic acid) and can by simple treatment with a mild alkali (for example sodium bicarbonate) be converted into e.g. sodium salt to acquire the negative charge. Then, they are reacted with glucosamine salt which is positively charged in order to form the complex. This complex is water-soluble and can be used to control the drug release from an aqueous solution.

The features disclosed in the foregoing description, in the claims and/or in the drawings may, both separately and in any combination thereof, be material for realizing the invention in diverse forms thereof.

## Claims

1. Composition comprising an ionic complex formed between an acidic pharmaceutical agent, or a salt or solvate thereof, and at least one complex-forming compound selected from the group consisting of glucosamine, chondroitin, hyaluronic acid and heparin, or a salt or derivative of one of these.

2. Composition according to claim 1, wherein the acidic pharmaceutical agent is selected from the group consisting of ibuprofen, diclofenac, valproate, gabapentin, cephalexin, cefixime and salts and solvates thereof.

3. Composition according to claim 1 or 2, wherein the molar ratio of the acidic pharmaceutical agent and complex-forming compound ranges from 1:10 to 10:1, preferably 1:1.

4. Composition according to any of claims 1 to 3, further comprising at least one water-soluble and/or water-dispersible diluent.

5. Composition according to any of claims 1 to 4, which is in liquid form.

6. Composition according to any of claims 1 to 5 for use in therapy.

7. Use of a composition according to any of claims 1 to 5 for preparation of a liquid controlled release oral drug delivery system.

## Amended claims

### Amended claims in accordance with Rule 86(2) EPC.

**1.** Use of a composition comprising an ionic complex formed between an acidic pharmaceutical agent, or a salt or solvate thereof, and at least one complex-forming compound selected from the group consisting of glucosamine, chondroitin, hyaluronic acid and heparin, or a salt or derivative of one of these, for preparation of a liquid controlled release oral drug delivery system.

**2.** Use according to claim 1, wherein the acidic pharmaceutical agent is selected from the group consisting of ibuprofen, diclofenac, valproate, gabapentin, cephalexin, cefixime and salts and solvates thereof.

**3.** Use according to claim 1 or 2, wherein the molar ratio of the acidic pharmaceutical agent and complex-forming compound ranges from 1:10 to 10:1.

**4.** Use according to claim 3, wherein the molar ratio is 1:1.

**5.** Use according to any of claims 1 to 4, wherein the composition further comprises at least one water-soluble and/or water-dispersible diluent.
